# EUROPÄISCHE PATENTANMELDUNG

(11) **EP 0 882 752 A2**
(43) Veröffentlichungstag der Anmeldung: **09.12.1998**
(21) Anmeldenummer: 98109414.7
(22) Anmeldetag: 23.05.1998
(51) Int. Cl.: C08G 77/26

(54) **2-Oxazolidinongruppen enthaltende Organosilane und Organopolysiloxane, deren Verwendung als grenzflächenaktive Substanzen sowie deren Verwendung zur Herstellung Amino- oder Carbamidgruppen enthaltender Organosilane und Organopolysiloxane**

(30) Priorität: 05.06.1997 DE 19723498
(71) Anmelder: Th. Goldschmidt AG, 45127 Essen (DE)
(72) Erfinder: Dietz, Thomas, Dr., 45259 Essen (DE); Weitemeyer, Christian, Dr., 45134 Essen (DE)

(57) **Zusammenfassung**

Die Erfindung betrifft Organosilane und Organopolysiloxane, die 2-Oxazolidinongruppen enthalten, wobei die Oxazolidinongruppe mit den Ring-Kohlenstoffatomen C-4 oder C-5 über einen Spacer an das Silan bzw. Siloxan geknüpft ist und der Spacer über eine hydrolysestabile Si-C-Bindung mit dem Silan bzw. Siloxan verbunden ist, deren Verwendung als grenzflächenaktive Substanzen sowie deren Verwendung zur Herstellung Amino- oder Carbamidgruppen enthaltender Organosilane und Organopolysiloxane.

## Beschreibung

Die Erfindung betrifft Organosilane und Organopolysiloxane, die 2-Oxazolidinongruppen enthalten, wobei die Oxazolidinongruppe mit den Ring-Kohlenstoffatomen C-4 oder C-5 über einen Spacer an das Silan bzw. Siloxan geknüpft ist und der Spacer über eine hydrolysestabile Si-C-Bindung mit dem Silan bzw. Siloxan verbunden ist, deren Verwendung als grenzflächenaktive Substanzen sowie deren Verwendung zur Herstellung Amino- oder Carbamidgruppen enthaltender Organosilane und Organopolysiloxane.

Oxazolidinonfunktionelle Silane und Polysiloxane weisen eine hohe Polarität auf, sind leicht herstellbar und finden aufgrund ihrer Grenzflächenaktivität und ihrer Affinität zu Oberflächen Verwendung als Additive für Dispersionsfarben und Lacke, zum Coaten von Oberflächen von Pigmenten und Füllstoffen oder in Körperpflege- und Körperreinigungsmitteln.

Zum Einsatz von Polysiloxanen in polaren Medien ist es erforderlich, polare Gruppen in das Siloxan einzubauen. Beispiele für polar modifizierte Siloxane sind Saccharid- (US-A-5 498 703), Carbonat- (DE-A-195 05 892), Imidazolin- (US-A-5 496 478) und Phosphatgruppen (US-A-5 530 084) enthaltende Polysiloxane. Die erfindungsgemäßen Verbindungen stellen eine neue Klasse organomodifizierter Polysiloxane mit besonderen Eigenschaften dar.

Es gibt eine Vielzahl von Methoden zur Herstellung von 2-Oxazolidinonen, wie sie in dem Übersichtsartikel von M. E. Dyen und D. Swern (Chem. Rev., 67, 1967, 197 - 246) beschrieben werden. Eine Methode geht von Epoxiden aus, die mit stickstoffhaltigen Reagenzien, wie Harnstoff, Alkylcarbamaten oder organischen Isocyanaten, umgesetzt werden: J. E. Herweh und W. J. Kauffman (Tetrahedron Letters, 12, 1971, 809 - 812) beschreiben die Lithiumbromid-katalysierte Reaktion von Epoxiden mit organischen Isocyanaten zu N-substituierten Oxazolidinonen in Kohlenwasserstoffen als Lösungsmittel. M. E. Dyen und D. Swern (J. Org. Chem., 33, 1968, 379 - 384) erhielten N-unsubstituierte Oxazolidinone bei der Umsetzung von Epoxiden mit Kaliumcyanat in Dimethylformamid unter Tetraethylammoniumbromid-Katalyse. K. Kamagata (Nippon Kagaku Kaishi, 11, 1985, 2073 - 2076) isolierte 5-(Phenoxymethyl)-2-Oxazolidinon bei der Reaktion von Phenylglycidether mit Isocyanursäure in Gegenwart von 2-Methylimidazol.

In DE-A-25 35 023 werden 5-(Aryloxymethyl)-2-Oxazolidinone beansprucht, deren Herstellung aus den entsprechenden Epoxiden mit Harnstoff sowie deren Verwendung als Arzneistoffe. A. Huth und F. Neubauer (Liebigs Ann. Chem. 1979, 56 - 62) fanden, daß sich bei der Reaktion mit Harnstoff die Ausbeuten erhöhen, wenn man Dimethylformamid als Lösungsmittel verwendet.

In EP-B-0 425 209 wird die Synthese von N-naphthylsubstituierten Oxazolidinonen aus Epoxiden mit N-Naphthylcarbamaten, in DE-A-26 30 107, GB-A-1 478 108, FR-B-2 281 114 und DE-A-29 02 129 die Synthese von N-unsubstituierten Oxazolidinonen aus Epoxiden mit Alkylcarbamaten wie Ethylcarbamat (Urethan) beschrieben. In EP-A-0 530 811 werden 5-(Alkoxymethyl)- oder 5-(Alkenyloxymethyl)-2-Oxazolidinone beansprucht sowie ein Verfahren zu deren Herstellung, bei dem unter Verwendung einer Kombination eines tertiären Amins mit einer Zinn-Verbindung als Katalysator das entsprechende Epoxid mit einem Carbamat ohne Lösungsmittel auf Temperaturen oberhalb 150 °C erhitzt wird.

In der Literatur ist zwar ein Organotrimethoxysilan (Recent Adv. Chiral Sep., [Proc. Chromatogr. Soc. Int. Symp. Chiral Sep.], 2nd (1990), Meeting Date 1989, 77 - 83. Editors: D. Stevenson, I. D. Wilson, Publisher: Plenum, New York, N. Y.) sowie ein monofunktionelles Organodisiloxan (Nippon Kagaku Kaishi, 5, 1990, 566 - 574 und Gan to Kagaku Ryoko, 7, 1980, 1942 - 1951) mit jeweils einer Oxazolidinongruppe beschrieben, in beiden Verbindungen trägt jedoch der Ringstickstoff einen Benzyl- bzw. Phenylsubstituenten. Es sind somit keine oxazolidinonfunktionellen Organosilane oder Organosiloxane, insbesondere Organopolysiloxane, bekannt, deren Oxazolidinon-Ring am Stickstoff einen Wasserstoff- oder kurzkettigen Alkylrest trägt. Durch die Abwesenheit aromatischer Substituenten am Stickstoff bleibt der polare Charakter der Oxazolidinongruppe erhalten und verleiht so den Verbindungen, insbesondere den von Natur aus unpolaren Polysiloxanen eine hohe Verträglichkeit mit polaren Medien.

Ein weiterer Vorteil der erfindungsgemäßen Polysiloxane im Vergleich zu dem bekannten monofunktionellen Disiloxan ist, daß das Verhältnis von (polaren) Oxazolidinon- und (unpolaren) Siloxangruppen beliebig eingestellt und so für die jeweilige Anwendung maßgeschneidert werden kann.

Es wurde nun gefunden, daß Organosilane und Organopolysiloxane, die Fünfring-Urethangruppen (2-Oxazolidinongruppen) enthalten, in einfachen Verfahren leicht herstellbar sind, günstige Eigenschaften in bevorzugt polaren Medien zeigen und zu amino- oder carbamidfunktionellen Silanen oder Polysiloxanen weiter umgesetzt werden können. Gegenstand der Erfindung sind demnach 2-Oxazolidinongruppen enthaltende Polysiloxane der allgemeinen durchschnittlichen Formel 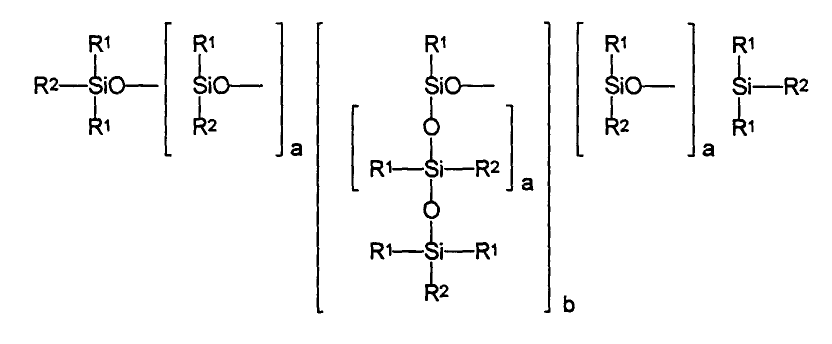 in der die Reste
- R¹: gleich oder verschieden sind und Alkylreste mit 1 bis 4 Kohlenstoffatomen oder Phenylreste bedeuten, mindestens 90 % der Reste R¹ aber Methylreste sind,
- R²: die Bedeutung der Reste R¹ haben können, aber mindestens ein Rest R² ein Rest der allgemeinen Formeln 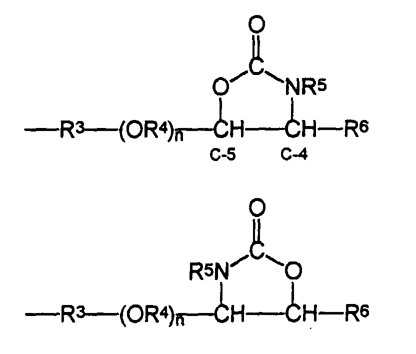 ist, in denen
- R³: ein gegebenenfalls verzweigter Alkylenrest mit 1 bis 20 C-Atomen ist und
- R⁴: gleiche oder verschiedene Alkylenreste mit 1 bis 4 C-Atomen bedeutet,
- R⁵: H oder ein Alkylrest mit 1 bis 8 C-Atomen ist,
- R⁶: H oder ein Alkylrest mit 1 bis 4 C-Atomen oder ein mit R³ einen Cyclus bildender Alkylenrest ist und
- n: einen Wert von 0 bis 20 hat,
- a: einen Wert von 0 bis 1000,
- b: einen Wert von 0 bis 10 hat.

Ein weiterer Gegenstand dieser Erfindung sind 2-Oxazolidinongruppen enthaltende Organosilane der allgemeinen Formel

R⁸₃₋ₘ(R⁸O)ₘSiR²

in der die Reste
- R⁸: gleich oder verschieden sind und Alkylreste mit 1 bis 4 Kohlenstoffatomen bedeuten,
- R²: ein Rest der oben angegebenen allgemeinen Formeln ist und
- m: einen Wert von 0 bis 3 hat.

Beispiele für den Rest R² (im Falle R² ≠ R¹) sind somit

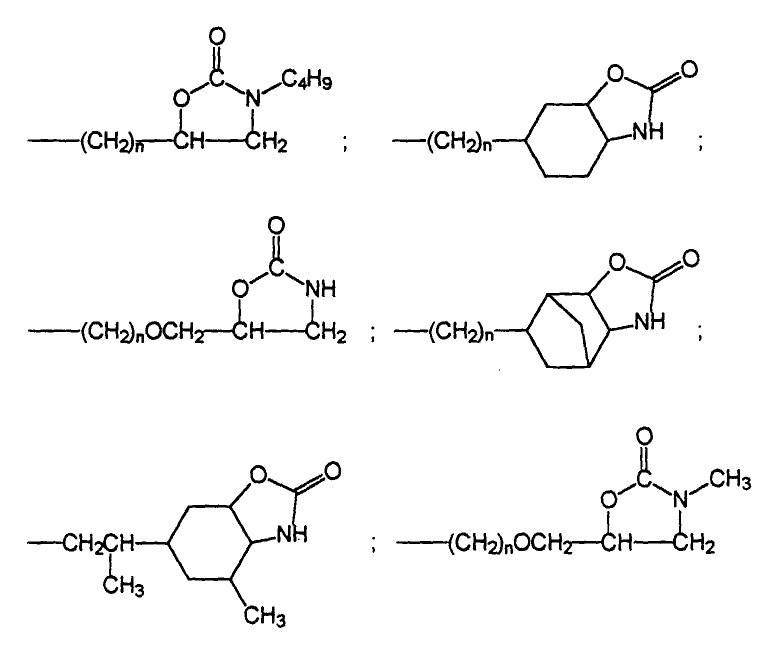

Als Beispiele für die erfindungsgemäßen Oxazolidinongruppen enthaltenden Silane und Polysiloxane seien genannt:

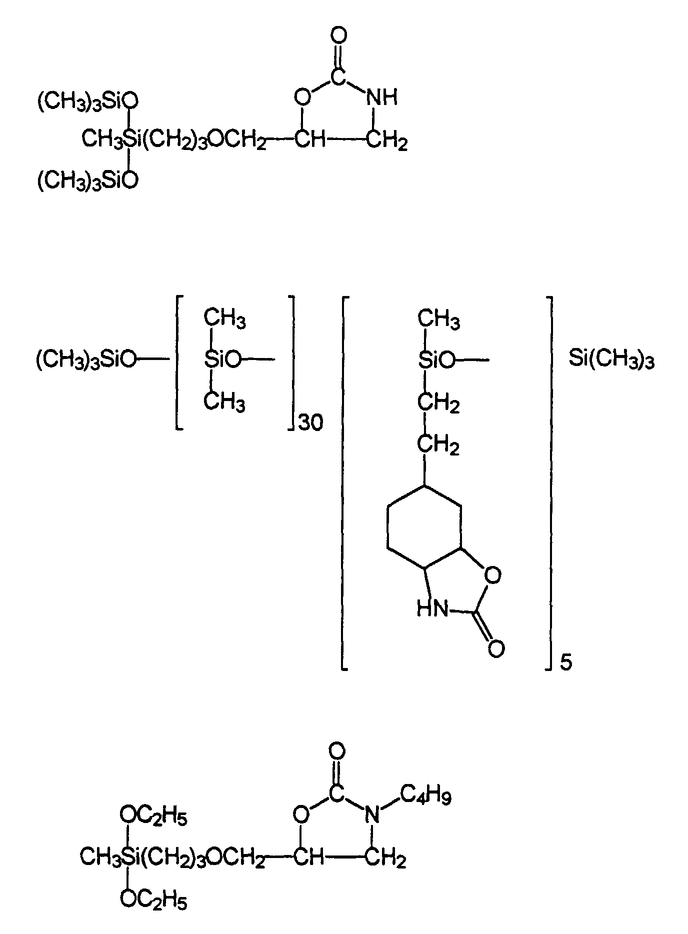

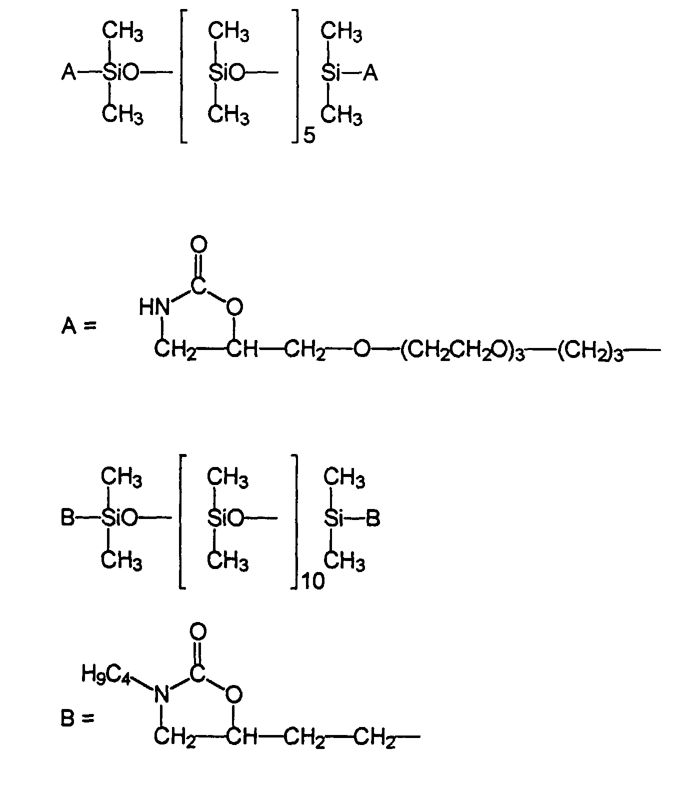

Die Herstellung Oxazolidinongruppen enthaltender Organosilane und Organopolysiloxane ist auf verschiedene Weise zugänglich. Ein Verfahren ist die Hydrosilylierung endständig C-C-Mehrfachbindungen enthaltender Oxazolidinone an ein Hydrogensilan oder Organohydrogenpolysiloxan. Oxazolidinonfunktionelle Mono-, Di- oder Trialkoxysilane können dann durch Hydrolyse und äquilibrierende Kondensation in ein Polysiloxan übergeführt werden. Bevorzugt ist jedoch ein Verfahren, bei dem von einem epoxyfunktionellen Silan oder Polysiloxan ausgegangen wird. Ein grundsätzliches Problem bei der Herstellung polar modifizierter Polysiloxane stellt nämlich der große Polaritätsunterschied zwischen dem (unpolaren) Siloxanrest und dem (polaren) organischen Rest dar. Dieses Problem tritt besonders dann auf, wenn eine C-C-Mehrfachbindungen enthaltende polare Verbindung in einer Hydrosilylierungsreaktion an ein Organohydrogensiloxan angelagert werden soll. Wegen der Unverträglichkeit der Reaktionspartner sind größere Mengen Lösungsmittel notwendig, was eine längere Reaktionsdauer und drastischere Reaktionsbedingungen zur Folge hat, was sich beides negativ auf die Reinheit der Produkte auswirkt. Oder man beobachtet gar keinen oder nur einen geringen Umsatz. Ein weiterer Nachteil der direkten Hydrosilylierung C-C-Mehrfachbindungen enthaltender Oxazolidinone ist, daß diese im Überschuß, bezogen auf das Organohydrogensiloxan, eingesetzt werden müssen, um einen möglichst vollständigen Umsatz zu erhalten. Dieser Überschuß kann im allgemeinen nicht destillativ entfernt werden und verbleibt somit im Produkt, wo er unter Umständen die vom oxazolidinonfunktionellen Siloxan gewünschten Eigenschaften negativ beeinflussen kann.

Es wurde nun überraschend gefunden, daß epoxyfunktionelle Polysiloxane mit einem stickstoffhaltigen Reagenz bei Temperaturen um 150 °C zu oxazolidinonfunktionellen Polysiloxanen umgesetzt werden können, ohne daß Abbaureaktionen am Siloxan beobachtet werden. Der Vorteil dieses bevorzugten und in den Beispielen beschriebenen Verfahrens liegt insbesondere darin, daß epoxyfunktionelle Organopolysiloxane aus dem Stand der Technik bekannt und kommerziell erhältlich sind. Ebenso ist es möglich, von Organopolysiloxanen auszugehen, die neben den epoxyfunktionellen Resten noch andere organische Reste tragen, z. B. Polyether- oder langkettige Alkylreste.

Entsprechend geht man aus von Verbindungen der allgemeinen durchschnittlichen Formel 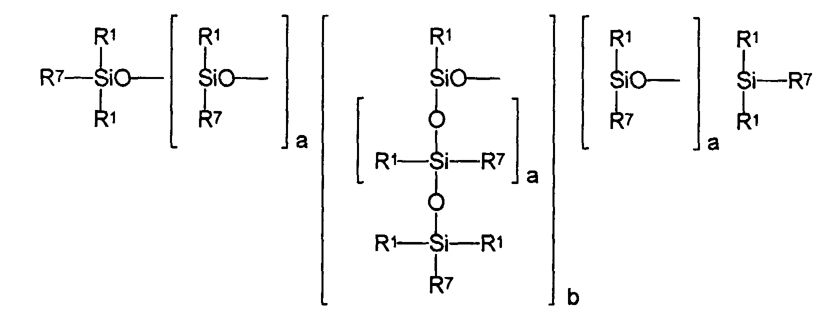 in der
- R⁷: R¹ sein kann, aber mindestens ein Rest R⁷ ein Rest der allgemeinen Formel 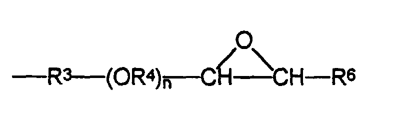 ist,
und die übrigen Reste und Indices die oben angegebene Bedeutung haben,
die man mit einem stickstoffhaltigen Reagenz umsetzt.

Die Umsetzung von Epoxiden zu 2-Oxazolidinonen mit verschiedenen stickstoffhaltigen Reagenzien ist in der Literatur beschrieben. Geeignete Reagenzien sind Harnstoff und dessen Derivate, N-substituierte und N-unsubstituierte Alkylcarbamate, Cyanamid, Cyanursäure, anorganische Cyanate und organische Isocyanate. Bevorzugt sind jedoch Harnstoff, N-unsubstituierte Alkylcarbamate sowie organische Isocyanate. Die Reaktion wird in Anwesenheit oder Abwesenheit eines Katalysators durchgeführt, bevorzugt jedoch in Anwesenheit eines Katalysators. Als Katalysatoren kommen Lewis-Säuren, Aminbasen, Organometallverbindungen, Übergangsmetallverbindungen sowie Alkali-, Erdalkali- oder Ammoniumsalze zum Einsatz, bevorzugt für die Reaktion mit epoxyfunktionellen Siloxanen sind jedoch diejenigen, die eine hohe katalytische Aktivität aufweisen und gleichzeitig die Bedingung erfüllen, daß durch sie keine Abbaureaktionen am Siloxangerüst induziert werden. In besonderem Maße eignen sich Übergangsmetallverbindungen, wie Alkyltitanate, z. B. Butyltitanat, oder Organozinnverbindungen, z. B. Dibutylzinndilaurat, sowie tertiäre Amine, z. B. Triethylamin. Als besonders geeignet erweist sich eine Kombination aus einem tertiären Amin und einer Organozinnverbindung, wie es in EP-A-0 530 811 beschrieben ist.

Die Reaktion kann in Anwesenheit oder Abwesenheit eines Lösungsmittels durchgeführt werden. Geeignete Lösungsmittel sind polare, hoch siedende Lösungsmittel, wie Dimethylformamid oder Ethylenglykoldimethylether. Bei Verwendung eines Lösungsmittels ist ein Verfahren besonders vorteilhaft, bei dem aus einem Alkohol, der gleichzeitig als Lösungsmittel dient, mit Harnstoff in Gegenwart eines geeigneten Katalysators das entsprechende Carbamat gebildet wird, das in situ oder in einer zweiten Stufe, jedoch in beiden Fällen in demselben Reaktionsgefäß mit dem epoxyfunktionellen Siloxan zum oxazolidinonfunktionellen Siloxan umgesetzt wird. Der Vorteil dieser Vorgehensweise besteht insbesondere darin, daß Harnstoff billiger und gesundheitlich unbedenklicher ist als bestimmte Alkylcarbamate. Ethylcarbamat zum Beispiel ist cancerogen eingestuft. Beispiele für geeignete Alkohole sind Butanol, Amylalkohol, Ethylenglykolmonomethylether, Methoxyisopropanol und Dipropylenglykolmonomethylether.

Die Reaktionen nach dem bevorzugten Verfahren werden üblicherweise bei Atmosphärendruck und Temperaturen zwischen 0 und 220 °C, vorzugsweise zwischen 70 und 160 °C, durchgeführt. Das stickstoffhaltige Reagenz wird in Verhältnissen von 0,5 bis 2,5 Mol, bezogen auf die Epoxyfunktion, eingesetzt, bevorzugt ist jedoch 1,0 Mol. Die Katalysatormenge liegt zwischen 0,1 und 5 Gew.-%, bezogen auf eingesetztes Silan- bzw. Siloxan-Reagenz-Gemisch, bevorzugt zwischen 0,5 und 3,0 Gew.-%. Falls ein Lösungsmittel verwendet wird, beträgt dessen Anteil am Gesamtansatz üblicherweise zwischen 30 und 70 Gew.-%. Die Reaktion wird bis zum vollständigen Umsatz des Epoxids durchgeführt, was NMR- oder IR-spektroskopisch bestimmt werden kann. Anschließend kann das eingesetzte Lösungsmittel oder während der Reaktion entstandene flüchtige Verbindungen, wie zum Beispiel Ethanol, das sich aus Ethylcarbamat bildet, abdestilliert werden. Bei Polysiloxanen bietet sich an, Lösungsmittel im Produkt zurückzulassen, da dadurch die Fließfähigkeit des Produkts gewährleistet bleibt.

Epoxysiloxane, bei denen die epoxyfunktionellen Reste über SiC-Bindungen mit den Si-Atomen des Siloxans verknüpft sind, werden in an sich bekannter Weise erhalten, indem man an SiH-Gruppen aufweisende Siloxane Epoxyalkene oder Epoxyalkenether, welche eine endständige, der Hydrosilylierung zugängliche Doppelbindung aufweisen, in Gegenwart von Hydrosilylierungskatalysatoren anlagert. Geeignete, kommerziell verfügbare Epoxyalkene oder Epoxyalkenether sind beispielsweise Limonenoxid, Allylglycidether, Vinylcyclohexenoxid und 3,4-Epoxy-1-buten.

Ein weiterer Gegenstand der Erfindung ist die Verwendung oxazolidinonfunktioneller Silane oder Polysiloxane als Zwischenprodukte zur Herstellung Amino- oder Carbamidgruppen enthaltender Silane oder Polysiloxane. Einen Überblick über mögliche Reaktionen von 2-Oxazolidinonen gibt der Übersichtsartikel von M. E. Dyen und D. Swern (Chem. Rev., 67, 1967, 197 - 246).

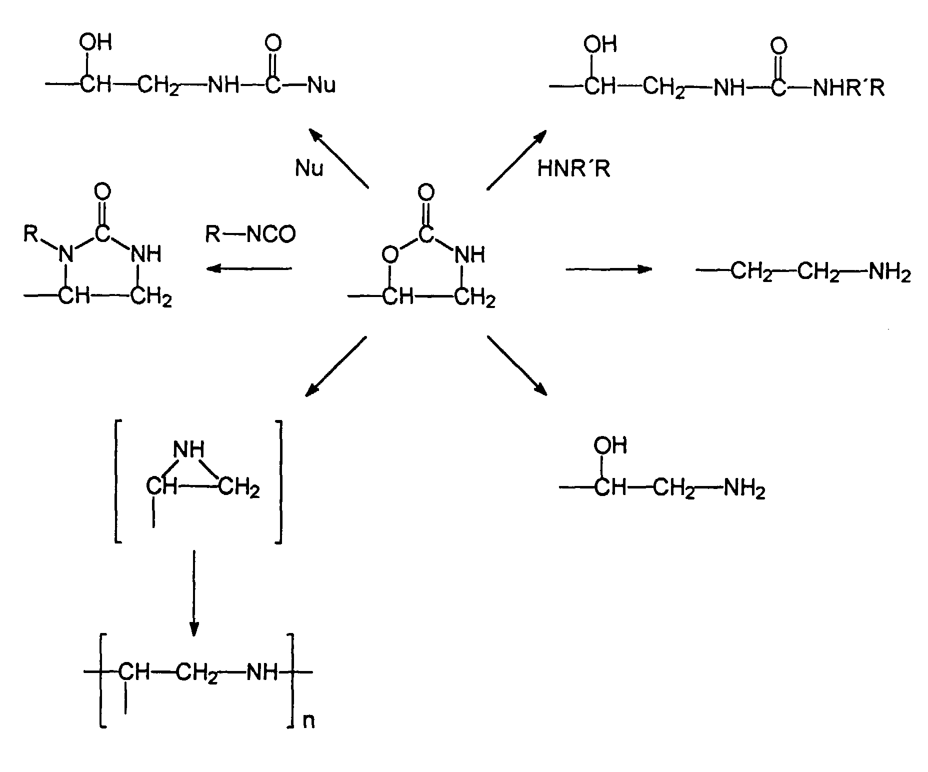

Oxazolidinone werden durch Reduktion oder Hydrolyse in die entsprechenden 2-Hydroxy-Amine übergeführt. Unter bestimmten Bedingungen ist eine desoxygenierende Reduktion zu Aminen möglich. Dies stellt einen neuartigen Zugang zu Aminosiloxanen dar, insbesondere zu solchen mit einem langen Spacer zwischen Aminfunktion und Siloxan-Rest. Aminosiloxane finden breite Anwendung, z. B. als Additive in Reinigungs- und Pflegemitteln. Zwar sind aminohydroxyfunktionelle Siloxane theoretisch aus epoxyfunktionellen Siloxanen durch Reaktion mit Ammoniak herstellbar, doch ist hierfür ein großer Überschuß an Ammoniak notwendig, der die Reaktion des Produkts mit noch nicht umgesetztem Epoxid unterdrücken soll. Diese Nebenreaktion kann gerade bei kammartigen Polysiloxanen zur Vernetzung und damit zu gelartigen Produkten führen. Der Vorteil der hier beschriebenen Zwei-Stufen-Synthese besteht darin, daß die oxazolidinonfunktionellen Siloxane ohne Nebenreaktion hergestellt werden können, aus denen dann die amino(hydroxy)funktionellen Siloxane freigesetzt werden können.

Durch nucleophile Öffnung des Oxazolidinon-Rings mit Stickstoffnucleophilen, wie Ammoniak, Hydrazin oder Aminen, erhält man eine 2-Hydroxyalkylharnstoffgruppe. Aber auch andere Nucleophile sind prinzipiell zur Derivatisierung geeignet.

Durch Reaktion mit organischen Isocyanaten werden Oxazolidinone zu N-substituierten Imidazolen umgesetzt.

Bei der Pyrolyse von Oxazolidinonen entstehen Aziridine bzw. deren Polymerisationsprodukte. Somit sind oxazolidinonfunktionelle Polysiloxane grundsätzlich geeignet zur Homopolymerisation zu siliconhaltigen Polyiminen oder zur Copolymerisation mit anderen Monomeren, z. B. Epoxiden oder Lactonen, was ihren Einsatz als Reaktivkomponente in der Kunststoffherstellung denkbar macht.

Ein weiterer Gegenstand der vorliegenden Erfindung ist die Verwendung der erfindungsgemäßen Verbindungen als grenzflächenaktive Substanzen. Eine wesentliche Bedeutung kommt der Funktionalität der Siloxancopolymeren, d. h. dem Verhältnis zwischen Oxazolidinon- und Siloxangruppen in den erfindungsgemäß zu verwendenden Organopolysiloxanen zu. Mit steigender Funktionalität nimmt der polare Charakter des Materials zu, und die Löslichkeit in polaren Lösungsmitteln steigt an. Beispiele für polare Lösungsmittel sind Wasser, wasserlösliche organische Lösungsmittel, wie Methanol, Ethanol, Isopropanol, Aceton, Dioxan, Dimethylformamid, Tetrahydrofuran, Dimethylsulfoxid sowie deren Gemische.

Auch durch die Alkylsubstituenten, die als Bindeglied zwischen dem Siloxangerüst und den Oxazolidinongruppen dienen, kann das polare Verhalten zusätzlich beeinflußt werden. Dabei zeigt sich, daß mit zunehmender Kettenlänge der Alkylreste auch der unpolare Charakter erhöht wird, wohingegen durch die Einführung von Polyetherresten die Löslichkeit in polaren Systemen zusätzlich erhöht wird.

Hierdurch wird dem Fachmann verständlich, daß die erfindungsgemäß zu verwendenden Organopolysiloxane zahlreiche Möglichkeiten bieten, die Polarität mit dem chemischen Charakter des eingesetzten Lösungsmittels, beispielsweise bis zur Wasserlöslichkeit, abzustimmen und deshalb in besonderer Weise dazu geeignet sind, sich dem jeweiligen Verwendungszweck anzupassen.

Die erfindungsgemäßen modifizierten Silane und Polysiloxane können somit in vielfältigen Anwendungen eingesetzt werden. Sie eignen sich besonders zum Einsatz in wäßrigen Medien, aus denen sie aufgrund ihrer Grenzflächenaktivität und ihrer Affinität zu Oberflächen ihre Wirkung entfalten. In Abhängigkeit von ihrem Aufbau können sie beim Einsatz in Lacken und Farben die Oberflächenbeschaffenheit der Beschichtung verbessern, sie können als Öl-in-Wasser- oder Wasser-in-Öl-Emulgatoren Einsatz finden oder beispielsweise in kosmetischen Präparaten zur Reinigung von Haut und Haaren, zur Verbesserung des Schaumes und zur Konditionierung der Haare bzw. zur Erzielung eines angenehmen Hautgefühls eingesetzt werden. Naturgemäß werden die erfindungsgemäßen modifizierten Polysiloxane häufig zusammen mit Tensiden und anderen Additiven zur Beeinflussung der Oberflächenbeschaffenheit eingesetzt. Alle genannten Formulierungen können die bekannten Zusatzstoffe enthalten, wie zum Beispiel:

Netzmittel, Tenside oder Emulgatoren aus den Klassen der anionischen, kationischen, zwitterionischen, amphoteren oder nicht-ionogenen oberflächenaktiven Substanzen, beispielsweise Fettalkoholsulfate, Fettalkoholethersulfate, Alkylsulfonate, Alkylbenzolsulfate, Sulfobernsteinsäurealkylester, quartäre Ammoniumsalze, Alkylbetaine, Carbonsäureamidoalkylbetaine, Derivate von monomeren oder höherkondensierten Sacchariden, oxethylierte Fettalkohole, Fettsäurealkanolamide oder oxethylierte Fettsäureester,
Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl.

Ferner ist ein Einsatz der erfindungsgemäßen Verbindungen als Textilhilfsmittel, als Additive in der Polyurethanschaumherstellung oder in der Formulierung von Additivpaketen für Treibstoffe denkbar.

### Herstellungs- und Verwendungsbeispiele

### Beispiel 1

209,7 g (0,1 Mol) eines seitenständig SiH-funktionellen Polydimethylsiloxans der allgemeinen Formel MD₂₀D^{H}_{7,5}M (Formel 1) und der mittleren Gesamtkettenlänge N = 29,5 werden mit 50 ml Toluol und 14,2 mg (= 20 ppm Pt) Hexachloroplatinsäure H₂PtCl₆ in einem 800-ml-Vierhalskolben, ausgestattet mit Rührer, Tropftrichter, Thermometer und Rückflußkühler, vorgelegt und unter Rühren auf 110 °C aufgeheizt. Bei dieser Temperatur werden 85,61 g (0,75 Mol) Allylglycidether derart zugetropft, daß trotz einsetzender exothermer Reaktion eine Temperatur von 130 °C nicht überschritten wird. Nach beendeter Zugabe wird die Reaktionsmischung noch 1 bis 2 h weiter bei 110 °C gerührt, bis die Umsatzkontrolle über den SiH-Wert zeigt, daß der Allylglycidether vollständig hydrosilylierend angelagert wurde. Bei einem Umsatz von > 99 % wird die Reaktion abgebrochen und die Pt-Katalysatorreste aus der Reaktionsmischung durch Filtration entfernt. Durch die Destillation im Ölpumpenvakuum werden Lösungsmittel und flüchtige Nebenprodukte entfernt.

28,9 g (0,01 Mol laut Epoxyzahl) des so hergestellten Epoxysiloxans (Formel 2) werden in einem mit Stickstoff gespülten Kolben, ausgestattet mit Rührer, Thermometer und Rückflußkühler, vorgelegt. 6,68 g (0,075 Mol) Ethylcarbamat (Urethan) werden darin durch Rühren gelöst und nach Zugabe von 0,71 g (= 2 %) Dimethylbenzylamin und 1,06 g (= 3 %) Dibutylzinndilaurat als Katalysatoren unter Rühren auf 150 °C erhitzt. Bei dieser Temperatur ist Rückfluß zu beobachten, der nach 1 h nur noch ganz schwach ist. Mittels ¹H-NMR-Spektroskopie kann der Umsatz anhand der Abnahme der Epoxidgruppe und der Zunahme der Oxazolidinongruppe bestimmt werden. Das Oxazolidinon besitzt als charakteristisches Signal ein Multiplett bei 4,7 ppm, das dem Proton an C-5 des Oxazolidinon-Rings zuzuordnen ist.

Nach 2 h wird auf Raumtemperatur abgekühlt. Nach der Destillation des während der Reaktion entstandenen Ethanols im Ölpumpenvakuum wird ein gelbes, zähflüssiges Reaktionsprodukt erhalten, welches seitenständige Oxazolidinongruppen aufweist und nach Analysenergebnissen der erwarteten mittleren Zusammensetzung MD₂₀D^{Oxazolidinon}_{7,5}M (Formel 3) entspricht. Läßt man das Ethanol im Produkt, so ist es besser fließfähig.

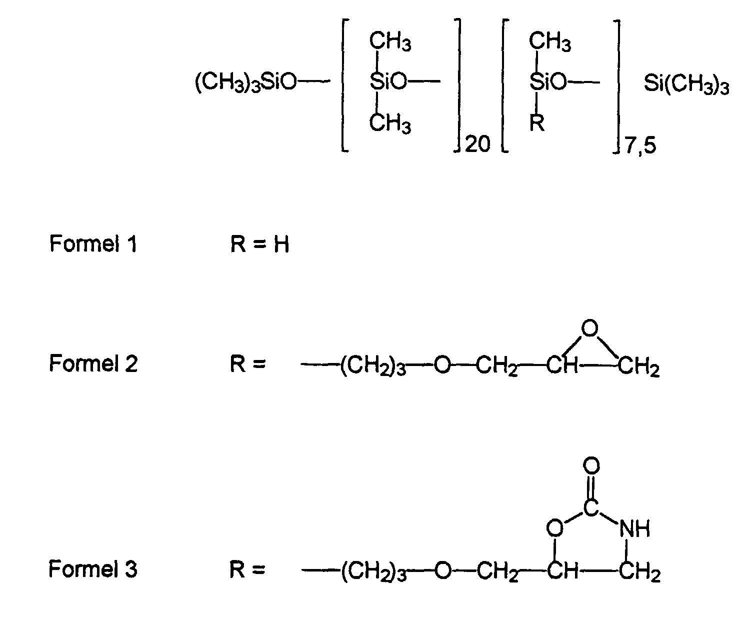

### Beispiel 2

In einem mit Stickstoff gespülten Kolben, ausgestattet mit Rührer, Thermometer, Tropftrichter und Rückflußkühler, werden 4,5 g (0,075 Mol) Harnstoff unter Rühren bei 135 °C geschmolzen. Bei dieser Temperatur wird eine Lösung von 28,9 g (0,01 Mol laut Epoxyzahl) des in Beispiel 1 hergestellten Epoxysiloxans in 35 ml Dimethylformamid während 1 h zugetropft und die Mischung weitere 7 h bei 135 °C gerührt. Nach Destillation des Lösungsmittels im Ölpumpenvakuum bei 100 °C erhält man ein gelbes, zähflüssiges Produkt, das analytisch mit dem in Beispiel 1 hergestellten Material identisch ist.

### Beispiel 3

In analoger Weise wie in Beispiel 1 dargelegt wird ein Epoxysiloxan der allgemeinen Formel M^{Epoxid}D₁₃M^{Epoxid} (Formel 4) und der mittleren Gesamtkettenlänge N = 15 durch die platinkatalysierte Anlagerung von 3,4-Epoxy-1-buten an ein entsprechendes SiH-Siloxan hergestellt.

In einem mit Stickstoff gespülten Kolben, ausgestattet mit Rührer, Thermometer, Tropftrichter und Rückflußkühler, werden 12,0 g (0,2 Mol) Harnstoff und 35,3 g (0,4 Mol) Amylalkohol mit 0,5 g (= 1 %) Butyltitanat als Katalysator 2 h unter Rühren auf 150 °C erhitzt. Anschließend gibt man 3,2 g (= 2 %) Dimethylbenzylamin und 4,8 g (= 3 %) Dibutylzinndilaurat als Katalysatoren zu und tropft 112,7 g (0,1 Mol laut Epoxywert) des oben hergestellten Epoxysiloxans (Formel 4) während 1 h bei 150 °C zu. Man läßt noch 3 h bei dieser Temperatur rühren und destilliert anschließend im Ölpumpenvakuum bei 100 °C das Lösungsmittel ab. Man erhält ein gelbes Reaktionsprodukt, welches endständige Oxazolidinongruppen aufweist und nach Analysenergebnissen der erwarteten mittleren Zusammensetzung M^{Oxazolidinon}D₁₃M^{Oxazolidinon} (Formel 5) entspricht.

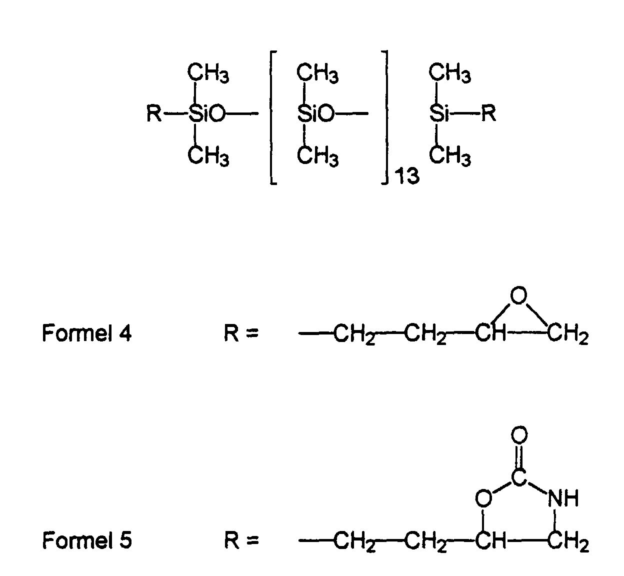

### Beispiel 4

Ein end- und seitenständig modifiziertes Epoxysiloxan der Formel M^{Epoxid}D₄D^{Epoxid}₄M^{Epoxid} (Formel 6) wird durch die Hydrosilylierung eines Polyoxyethylenpolymeren der mittleren Durchschnittsformel CH₂=CH-CH₂-(O-C₂H₄)₇-OH an das entsprechende SiH-Siloxan und der daran anschließenden Endverkappung der primären Hydroxygruppen des Siloxancopolymeren mit Epichlorhydrin erhalten. In einer nachfolgenden Reaktion werden in einem mit Stickstoff gespülten Kolben, ausgestattet mit Rührer, Thermometer und Rückflußkühler, 323,1 g (0,1 Mol laut Epoxywert) dieses Epoxysiloxans zusammen mit 36,1 g (0,6 Mol) Harnstoff, 91,3 g (1,2 Mol) Ethylenglykolmonomethylether sowie 4,5 g (= 1 %) Isopropyltitanat, 9,0 g (= 2 %) Dimethylbenzylamin und 13,5 g (= 3 %) Dibutylzinndilaurat als Katalysatoren vorgelegt und die Mischung unter Rühren 5 h auf 150 °C erhitzt. Nach Destillation des Lösungsmittels im Ölpumpenvakuum bei 100 °C erhält man ein gelbes Reaktionsprodukt, welches end- und seitenständige Oxazolidinongruppen aufweist und nach Analysenergebnissen der erwarteten mittleren Zusammensetzung M^{Oxazolidinon}D₄D^{Oxazolidinon}₄M^{Oxazolidinon} (Formel 7) entspricht.

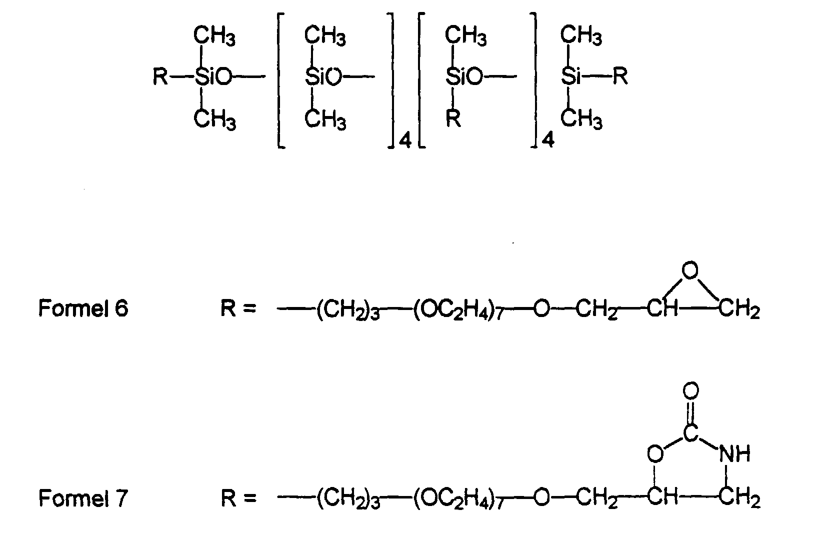

### Beispiel 5

In einem mit Stickstoff gespülten Dreihalskolben, ausgestattet mit Rührer, Thermometer und Rückflußkühler, werden 24,8 g (0,1 Mol) käuflich erwerblichen Glycidoxypropylmethyldiethoxysilans (Formel 8) vorgelegt. 8,9 g (0,1 Mol) Ethylcarbamat werden durch Rühren darin gelöst und nach Zugabe von 0,67 g (= 2 %) Dimethylbenzylamin und 1,01 g (= 3 %) Dibutylzinndilaurat als Katalysatoren unter Rühren 1 h auf 120 °C erhitzt. Anschließend destilliert man entstandenes Ethanol über eine Destillationsbrücke bei Normaldruck ab. Man erhält ein gelbes Reaktionsprodukt, das nach Analysenergebnissen die erwartete Strukur (Formel 9) besitzt.

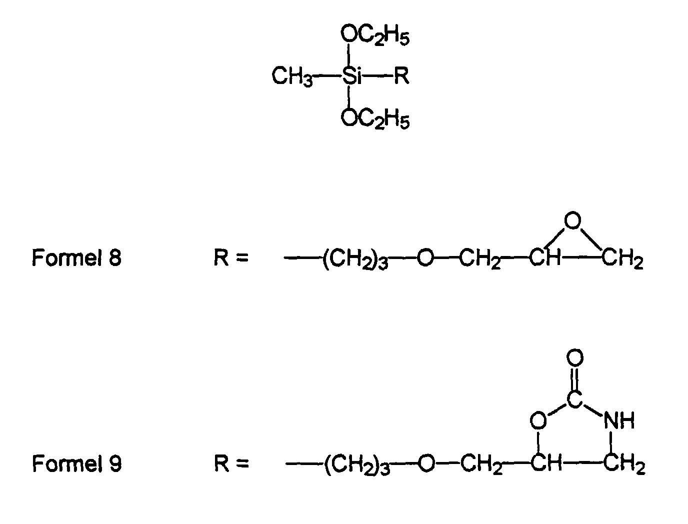

### Beispiel 6

In einem mit Stickstoff gespülten Kolben, ausgestattet mit Rührer, Thermometer, Tropftrichter und Rückflußkühler, wird unter Rühren eine Lösung von 28,9 g (0,01 Mol laut Epoxyzahl) des in Beispiel 1 hergestellten Epoxysiloxans und 1,1 g (= 3 %) Lithiumchlorid in 60 ml Dimethylformamid auf 150 °C erhitzt. Während 1 h tropft man eine Lösung von 7,4 g (0,075 Mol) Butylisocyanat in 15 ml Dimethylformamid zu und rührt die Mischung weitere 3 h bei 150 °C. Nach Destillation des Lösungsmittels im Ölpumpenvakuum bei 100 °C erhält man ein hellbraunes Produkt, das nach Analysenergebnissen die erwartete Struktur (Formel 10) besitzt.

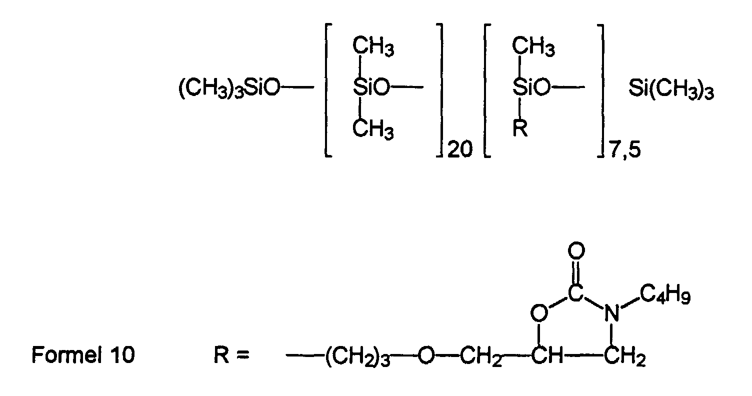

### Anwendungstechnische Prüfungen

Zur Überprüfung der anwendungstechnischen Eigenschaften der erfindungsgemäßen oxazolidinonfunktionellen Polysiloxane wird das Produkt aus Beispiel 1 in jeweils 1 %iger Konzentration mit diversen Lösungsmittel-Wasser-Gemischen abgemischt. In den nachfolgenden Tabellen sind die erhaltenen Ergebnisse im Vergleich mit dem nicht erfindungsgemäßen Ausgangsmaterial angeführt. Man sieht anhand der Ergebnisse, daß die oxazolidinonfunktionellen Polysiloxane einen deutlich polareren Charakter haben als die entsprechenden epoxyfunktionellen Siloxane. Im Falle eines 3 : 1-Mischungsverhältnisses von Lösungsmittel zu Wasser ergibt das oxazolidinonfunktionelle Siloxan in allen Fällen eine homogene Lösung, während das epoxyfunktionelle Siloxan in den überwiegenden Fällen zu ölartigen Abscheidungen führt.

**Tab. 1**

| Aussehen 1 %iger Lösungen eines oxazolidinonfunktionellen Siloxans (M-D₂₀-D^{Oxazolidinon}_{7,5}-M) in Lösungsmittel-Wasser-Gemischen (1 : 1) im Vergleich zu Lösungen des entsprechenden epoxyfunktionellen Siloxans | | |
|---|---|---|
| Lösungsmittel des 1 : 1-Gemischs mit Wasser | Aussehen¹⁾ 1 %iger Lösungen von Siloxan- | |
| | Oxazolidinon | Epoxid |
| Isopropanol | 1 | 3 |
| Ethanol | 2 | 3 |
| Dioxan | 2 | 3 |
| Methanol | 3 | 3 |
| Aceton | 3 | 3 |
| Tetrahydrofuran | 3 | 3 |
| Ethylenglykolmonomethylether | 3 | 3 |
| Propylenglykol | 3 | 3 |

| | | |
|---|---|---|
| ¹⁾ 1 = klare Lösung 2 = trüb oder opak, aber homogen 3 = Niederschlag bzw. ölige Abscheidung | | |

**Tab. 2**

| Aussehen 1 %iger Lösungen eines oxazolidinonfunktionellen Siloxans (M-D₂₀-D^{Oxazolidinon}_{7,5}-M) in Lösungsmittel-Wasser-Gemischen (3 : 1) im Vergleich zu Lösungen des entsprechenden epoxyfunktionellen Siloxans | | |
|---|---|---|
| Lösungsmittel des 3 : 1-Gemischs mit Wasser | Aussehen¹⁾ 1 %iger Lösungen von Siloxan- | |
| | Oxazolidinon | Epoxid |
| Tetrahydrofuran | 1 | 2 |
| Isopropanol | 1 | 3 |
| Ethanol | 1 | 3 |
| Aceton | 1 | 3 |
| Ethylenglykolmonomethylether | 2 | 2 |
| Dioxan | 2 | 2 |
| Methanol | 2 | 3 |
| Propylenglykol | 2 | 3 |

| | | |
|---|---|---|
| ¹⁾ 1 = klare Lösung 2 = trüb oder opak, aber homogen 3 = Niederschlag bzw. ölige Abscheidung | | |

Aus den Tabellen ergibt sich somit, daß die erfindungsgemäß zu verwendenden modifizierten Organopolysiloxane die gewünschten anwendungstechnischen Eigenschaften besitzen.

## Patentansprüche

1. 2-Oxazolidinongruppen enthaltende Polysiloxane der allgemeinen durchschnittlichen Formel 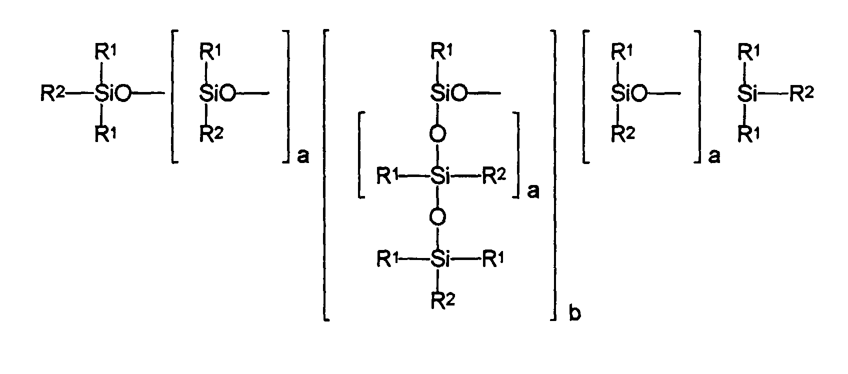 in der die Reste
R¹ gleich oder verschieden sind und Alkylreste mit 1 bis 4 Kohlenstoffatomen oder Phenylreste bedeuten, mindestens 90 % der Reste R¹ aber Methylreste sind,
R² die Bedeutung der Reste R¹ haben können, aber mindestens ein Rest R² ein Rest der allgemeinen Formeln 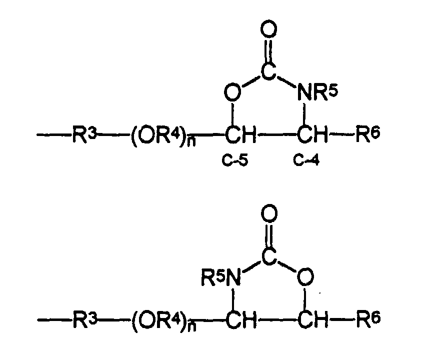 ist, in denen
R³ ein gegebenenfalls verzweigter Alkylenrest mit 1 bis 20 C-Atomen und
R⁴ gleiche oder verschiedene Alkylenreste mit 1 bis 4 C-Atomen,
R⁵ H oder ein Alkylrest mit 1 bis 8 C-Atomen,
R⁶ H oder ein Alkylrest mit 1 bis 4 C-Atomen oder ein mit R³ einen Cyclus bildender Alkylenrest ist und
n einen Wert von 0 bis 20 hat,
a einen Wert von 0 bis 1000,
b einen Wert von 0 bis 10 hat,
oder
2-Oxazolidinongruppen enthaltende Organosilane der allgemeinen Formel
R⁸_{3 - m}(R⁸O)ₘSiR²
in der die Reste
R⁸ gleich oder verschieden sind und Alkylreste mit 1 bis 4 Kohlenstoffatomen bedeuten,
R² ein Rest der oben angegebenen allgemeinen Formeln ist und
m einen Wert von 0 bis 3 hat.

2. Verwendung der Verbindungen gemäß Anspruch 1 als grenzflächenaktive oder Affinität zu Oberflächen aufweisende Substanzen in bevorzugt polaren, insbesondere wäßrigen Medien.

3. Verwendung der Verbindungen gemäß den Anspruch 1 in Kombination mit Tensiden und anderen Additiven zur Beeinflussung der Oberflächenbeschaffenheit.
